# EUROPEAN PATENT APPLICATION

(11) **EP 4 011 418 A1**
(43) Date of publication of application: **15.06.2022**
(21) Application number: 20212852.6
(22) Date of filing: 09.12.2020
(51) Int. Cl.: A61M 3/00, A61M 31/00, A61H 35/04

(54) **DEVICE FOR CONTROLLED ADMINISTRATION OF A LIQUID SOLUTION TO NASAL CAVITIES AND PARANASAL SINUSES**

(71) Applicant: NAOUM, George, 11475 Gkizi Attikis (GR)
(72) Inventor: NAOUM, George, 11475 Gkizi Attikis (GR)
(74) Representative: Gallego Jiménez, José Fernando

(57) **Abstract**

Devices for controlled administration of liquid solutions to nasal cavities and paranasal sinuses are disclosed. The proposed devices comprise an external housing (1) defining an inner space and having a proximal and a distal end. The external housing comprises an air system (2) for injecting air to a nostril plugs assembly (9); a pump system (3) for pumping the liquid solution to the nasal cavities; a removable cartridge (4) holding the liquid solution, connected to the pump system; and a nostril plugs system (5) removably connected to the pump system. The nostril plugs system comprises a two-line resilient tube (7); a plug-in system (8) connected to the two-line resilient tube; and a nostril plugs assembly connected to the other end of the resilient tube. The proposed devices may be used for the treatment of respiratory diseases including the respiratory syndrome coronavirus 2 (SARS-CoV-2).

## Description

### FIELD OF THE INVENTION

The present invention relates to a device for controlled administration of a liquid solution to the nasal cavities and paranasal sinuses of a user. The device is intended to be used for the cleaning and / or disinfection of nasal cavities and paranasal sinuses and / or for the treatment of nasal and / or sinus disorders such as sinus inflammation, including nasal congestion.

### BACKGROUND

Sinus and nasal congestion or rhinitis, involves the blockage of one or more of the paranasal sinus passageways in the skull. The paranasal sinuses are air-filled cavities within the facial skeleton. Each paranasal sinus is contiguous with a nasal cavity and connects with it via small orifices called ostia. The blockage of the ostia may result from inflammation and swelling of nasal tissues or the presence of excess mucus. When inflammation occurs, normal drainage of mucus from within the sinuses is disrupted, and sinusitis may occur.

Described paranasal sinuses are frontalis sinuses, ethmoid sinuses, sphenoid sinuses and maxillary sinuses.

While acute nasal congestion is frequently caused by acute infections, chronic nasal congestion usually results from exposure to irritants such as tobacco smoke, food allergens, inhaled allergens, or foreign matter within the nasal cavity. So, maintenance of clear and healthy nasal cavities and paranasal sinuses of humans is part of proper medical, family and / or personal care.

Further, the respiratory system is subject to continuous risks of viral diseases and microbial infections, mainly during the winter. SARS-Cov2 infection mainly begins in the nasopharyngeal area, it is incubated there for a period of time and then it gains entrance to the rest of the respiratory tract^{1,2}. Hypertonic saline exposure of human cells like epithelial, mucosal and immunity cells in the upper respiratory system increases their antiviral activity against many respiratory pathogens including previously known coronaviruses and SARS-Cov2. Specifically, hypertonic saline solution promotes innate immunity responses in various ways, such as inducing depolarization of the epithelial cells putting them in a low energy state, thus decreasing the ability of the virus to replicate³ and / or it induces increased production of hypochlorite ions via myeloperoxidase dependent reaction due to increased chloride ions concentration⁴. This increase in antiviral innate immunity has been shown clinically in upper respiratory infection including other coronaviruses where the use of hypertonic saline has reduced both the duration of disease and the need for use of other medications⁵.

Traditional techniques to clean the nasal cavities and to treat nasal congestion include techniques of nasal irrigation and use of decongestant sprays which cause vasoconstriction of the nasal cavity mucosa reducing inflammation.

Decongestant sprays rapidly reduce edema mainly in the nasal cavity mucosa and make breathing through the nose easier. However, these sprays do not reach the whole mucous membrane surface of the nasal cavities and the paranasal sinuses. It is needed to reach these parts of the mucosa to treat illnesses, e.g. sinusitis, which cannot be cured by current limited local treatment.

Another way of administering liquid solutions into the nasal cavities is by allowing a solution to flow through a tightening olive, which fits to the nostril, on its upper side and can be attached to some kind of container or tube on its bottom. Although the olive fits the nostril, it is not possible to fill the nose and let the solution reach the upper and inner parts of the nose, as the solution runs back into the pharynx along the bottom of the cavities.

Researchers and medical device manufacturers, acknowledging the great benefits to humans of having a healthy respiratory system, continuously try to find ways to inject therapeutic solutions to the paranasal sinuses. Many existing devices use various techniques but only partially achieve cleaning of the nostrils from excretions and do not succeed in injecting therapeutic solution to the paranasal sinuses in a controlled way.

For example, the device described in US 2014/0121592 provide liquid in the nasal cavities, allowing for a quick cleaning or short-term treatment of the nasal cavities. However, the infected fluid returns to the container from the same spout, probably passing some infected material to the clean liquid. This short term period leads to a temporary solution.

In addition, the device of WO 2013/072856 has to be connected to a source of compressed air in order to provide the fluid in the nostril. If the user was to receive a treatment in the paranasal cavities, the use of such a device would either cause the user to choke and/or to feel highly uncomfortable, stressed, while injury could be provoked to upper respiratory system including ears via eustcahian tubes.

In addition, WO 99/26684 describes a device designed for delivering a constant controlled flow of liquid or many equal doses of liquid to the nasal cavities. However, even this device, is used for rinsing the nasal cavities and does not allow for the liquid to enter and remain in the nasal cavities and paranasal sinuses and thus does not face the root of the problem. Although this device is supposed to provide liquid in a more controllable way, if it is to be used with a delivering probe, like the one described in US 2017/0340869 which blocks the nostrils in order for the liquid to reach the paranasal cavities, the continuous flow would probably cause choking to the user.

Consequently, new devices for dispensing a liquid solution, which make it possible to fill the nasal cavities with a liquid solution, thereby reaching the paranasal sinuses and covering the whole nasal mucous membrane surface while avoiding the solution to run back into the pharynx, are desirable.

### SUMMARY

The proposed device hereof may provide for cleaning and / or disinfecting the nasal cavities and paranasal sinuses and may also achieve injection of a liquid solution to the paranasal sinuses for the treatment of sinus infections.

To this aim, according to an example, the device for controlled provision of a liquid solution to nasal cavities and paranasal sinuses of a user, is comprising:
- an external housing defining an inner space and having a proximal end and a distal end, the external housing comprising:
- an air system for injecting air to a nostril plugs assembly;
- a pump system for pumping the liquid solution to the nasal cavities and connected to the air system by means of a resilient tube;
- a cartridge holding the liquid solution and removably connected to the pump system at the proximal end of the external housing; and
- a nostril plugs system removably connected to the pump system at the distal end of the external housing and comprising:
   - a two-line resilient tube having a proximal end and a distal end;
   - a plug-in system connected to the proximal end of the resilient tube; and
   - a nostril plugs assembly connected to the distal end of the resilient tube.

The device is manual and has no electronic or electrical components. It has a special design to give the user the appropriate ergonomics while it is reusable with the use of different cartridges.

The external housing of the device is designed to offer significant time saving of assembly, it is made of a plastic material, it does not contain third-party materials, such as glue or screws, and holds the air system and the pump system through a snap-fit system having a subframe that closes all components internally.

The ergonomic design of the external housing provides proper driving of the cartridge and allows for correct use of an air bulb, comprised within the air system and located at the bottom of the device, to inject air at the beginning of the treatment.

The device is intended to initially inject air, or other fluid or gas, into the nostril plugs system, and hold the air, by means of a one-way valve, so that two expandable portions of a nostril plugs component, comprised within the nostril plugs system and having a network of internal tubes, remain inflated and block the nose and the user from breathing. Then, by mechanical movement of the cartridge, the pump system is forced to channel the liquid solution and fill with said solution the nasal cavities.

By means of a two-line resilient tube that has an air line and a liquid line, the device is able to inject air coming from the air system to the nostril plugs component, creating an air-tight seal with the nostrils, and to pump the liquid solution, through the nostril plugs system and into the nasal cavities.

The expandable portions of the nostril plugs component are designed to be bell-shaped to lock into the inner cavity as soon as they are placed on the nose. An important part of the nostril plugs component in its design is the angle between the two bell-shaped expandable portions so as to offer perfect adaptation and ergonomics to the human nose but also offer comfort and safety in the nasal cavities.

The nostril plugs system is connected to the pump system by means of a plug-in mechanism which makes the attachment and detachment of the nostril plugs system to the device easier for the user.

The use of such a device makes easier the delivery of liquid to the paranasal sinuses, since the expandable portions of the nostrils plug component are fitted in the nasal cavities blocking them and not allowing to the fluid to exit the nose. Treatment or cleaning of the paranasal sinuses cannot be delivered simply by rinsing the nasal cavities, and requires an advanced quantity of fluid to be held in the nasal cavities, which is accomplished by prohibiting the fluid to exit the nasal cavities until the treatment is completed.

The cartridge comprises a lid which is locked with a container that holds a liquid solution and all the components are insulated internally ensuring airtight operation and protection from the environment. The cartridge also comprises a safety mechanism to protect the connection area of the cartridge from dirt and to certify that the cartridge has not been used before. By breaking the safety mechanism, the cartridge is ready for use until the whole solution is emptied and cannot be reused or refilled again by the user.

The operation of the cartridge is based on the suction of the pump system. When the cartridge is connected to the pump system, a watertight chamber is created which also acts as a dosimeter of the solution during use. The pump system pumping solution from the cartridge forces a piston comprised within the container of the cartridge to move upwards due to the vacuum created inside the container and a one-way valve mechanism, also comprised within the container, doesn't allow the liquid solution to return to the container.

The container may comprise a saline solution for bathing the nasal and sinus cavities to wash away encrusted mucous, irritants, and foreign particles for the purpose of improving airflow and relieving nasal congestion. However, the container may also contain other liquid solutions, such as medicinal solutions, i.e. antibiotic solutions, for the upper respiratory system or liquids containing extracts of herbal plants which may also or alternatively be beneficial for the upper respiratory system.

### BRIEF DESCRIPTION OF THE DRAWINGS

Non-limiting examples of the present disclosure will be described in the following, with reference to the appended drawings, in which:
FIG. 1 is an exploded view of the main components of a device according to an example of the present invention.
FIG. 2 is a perspective view of the connection between an air system and a pump system according to an example of the present invention.
FIG. 3 is a sectional view of a pump system according to an example of the present invention.
FIG. 4 is a sectional view of an air system according to an example of the present invention.
FIG. 5 is a sectional view of a cartridge according to an example of the present invention.
FIG. 6 is an exploded view of the cartridge of FIG.5.
FIG. 7 shows the connection between a cartridge and a pump system according to an example of the present invention.
FIG. 8 shows the connection between a pump system and a nostril plug system according to an example of the present invention.
FIG. 9 shows an exploded view of nostril plug system according to an example of the present invention.
FIG. 10 is a sectional view of nostril plugs component according to an example of the present invention.
FIG. 11 is an exploded view of a device according to the present invention.

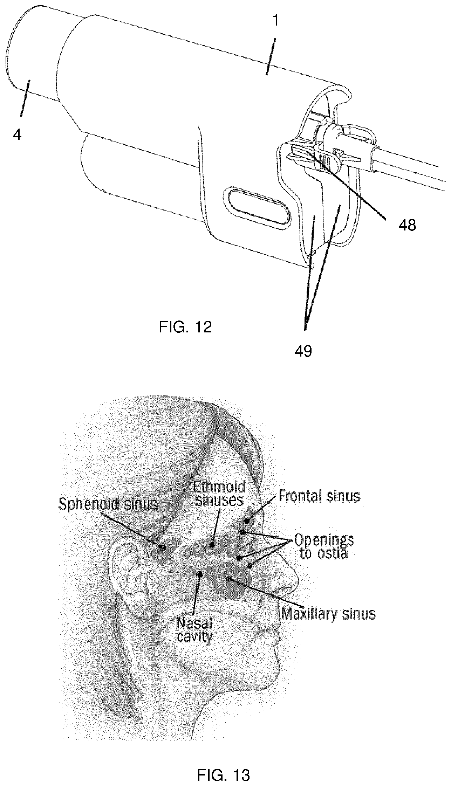

FIG. 12 shows the snap-fit mechanism and subset frame of an external housing according to an example of the present invention.
FIG.13 is a schematic representation of anatomy of the nasal cavities and paranasal sinuses.
FIG. 14a-14c are representations of the positioning of the nostril plugs assembly by a user.

### DETAILED DESCRIPTION OF THE INVENTION

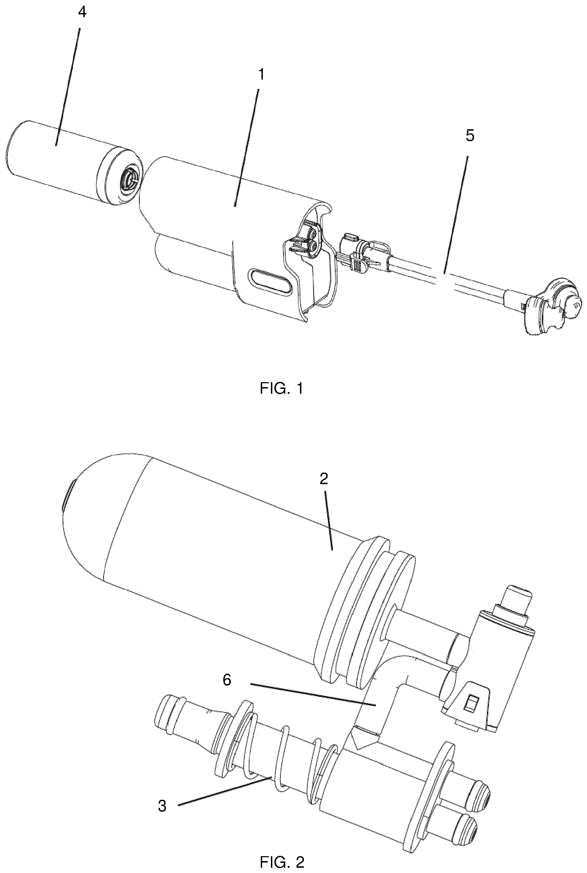

FIG. 1 is an exploded view of a device for provision of a liquid solution to the nasal cavities and paranasal sinuses of a user according to an example of the present invention. As shown in FIG. 1, the device comprises an external housing 1; a cartridge 4 holding a liquid solution; and a nostril plugs system 5 for delivering the liquid solution to the nasal cavities and paranasal sinuses of a user.

The external housing 1 defines an inner space comprising an air system 2 for injecting air to a nostril plugs assembly 9 and a pump system 3 for pumping a liquid solution to the nasal cavities.

According to a preferred embodiment of the present invention, the external housing 1 is made of a plastic material.

As shown in FIG. 2, the air system 2 and the pump system 3 are connected to each other by means of a resilient tube 6 and held together inside the external housing 1 by means of a snap-fit mechanism 48 through a sub-frame 49 that holds both systems firmly in place without the need for screws.

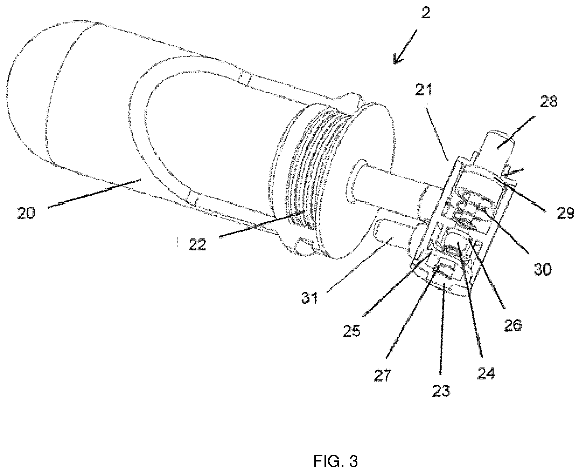

FIG.3 shows an air system 2 according to a preferred embodiment of the present invention, wherein the air system 2 comprises an air bulb 20 and an air release valve 21 connected to the air bulb 20 through an air bulb base 22. The air release valve 21 comprises a valve cover 23; a valve cover sealing means 25; a valve plunger 24; a valve plunger sealing means 26; a first spring means 27 that applies a constant pressure holding the valve plunger in position with the valve plunger sealing means, thus sealing and allowing air flow in only one direction, preventing reverse flow; an actuator 28 activated by mechanical actuation of a user's pressure, an actuator sealing means 29; a second spring means 30 that pushes the valve plunger to allow air release and to return the actuator 28 to its original position; and an air outlet 31 for connection to the resilient tube 6. When the actuating force is removed, the spring returns the valve to its original position.

In a preferred embodiment, the valve plunger 24 is shaped like a ball and the actuator sealing means 29 is an O-ring.

In a preferred embodiment, all of the air system 2 components are made of plastic and have a special design that aims to create the right position for the air bulb 20 and the actuator 28 to help the user ergonomically during the treatment.

The air system 2 is built in such a way that the device doesn't comprise any electronic or electrical parts and is completely autonomous.

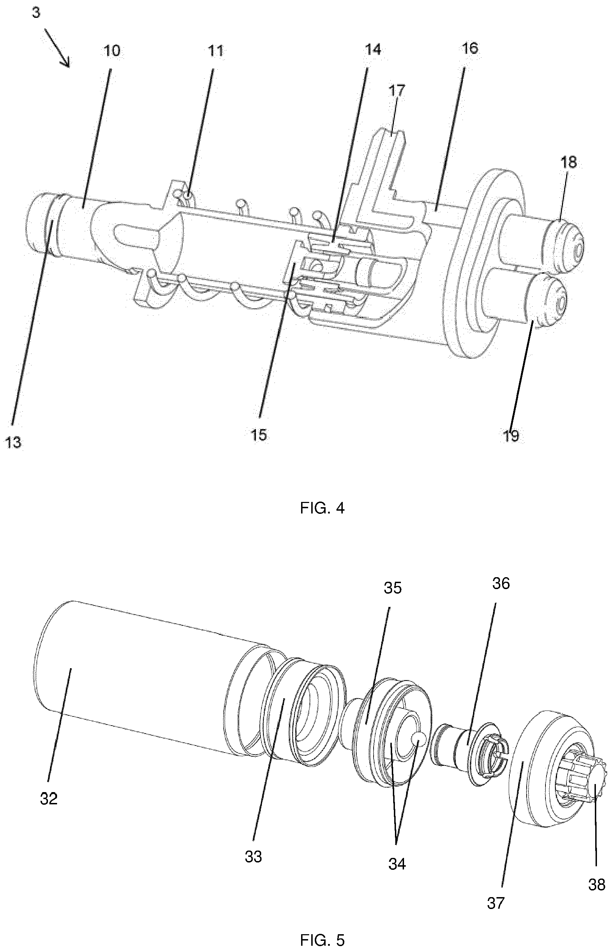

FIG. 4 shows a pump system 3 according to a preferred embodiment of the present invention, the pump system 3 comprising a cylinder 10; a spring means 11 arranged around the outside of the cylinder 10; a hollow piston 14 which is movable in the cylinder 10; a valve member 15 which is guided by the hollow piston and is mounted so as to be axially movable with the piston 14; a pump nozzle 12 for connection with the cartridge 4 comprising sealing means 13; and a pump base 16 comprising an air inlet port 17 connected to an air discharge plug 18 to channel the air coming from the air system and a liquid discharge plug 19 to channel the liquid from the cartridge 4.

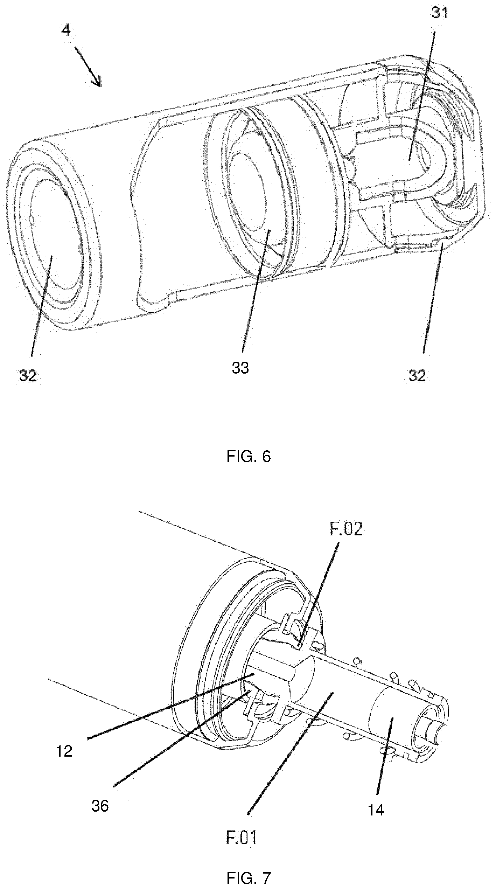

As shown in FIGs. 5 and 6, in a preferred embodiment of the present invention, the cartridge 4 comprises a container 32; a hollow cylindrical piston 33 arranged inside the container 32; a one-way valve mechanism 34; a pump hose with airtight connection 36; a container lid 35 to lock the container 32 and a cartridge lid 37.

The cartridge lid 37 comprises a safety lid 38 to protect the connection area of the cartridge from dirt and to certify that the cartridge has not been used before.

The container 32 holds approximately between 50ml and 150 ml, which according to medical research is the quantity of fluid required to fill both the nasal and the paranasal cavities of a user or patient. Therefore, this is the required quantity of fluid to deliver a proper treatment of this kind.

In a preferred embodiment, the container holds 50ml of a liquid solution.

As shown in FIG. 7, the cartridge 4 is removably connected to the pump system at the proximal end of the external housing 1.

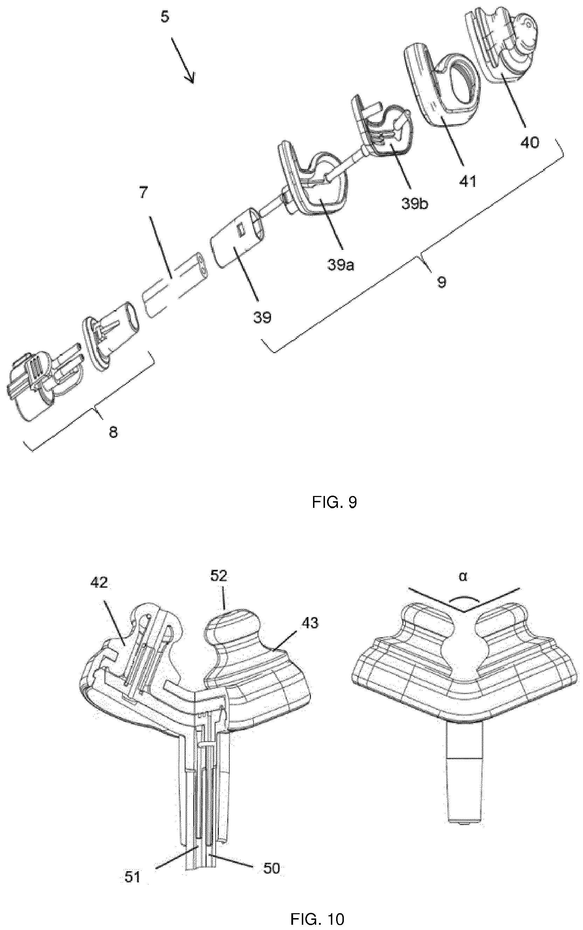

As shown in FIG.9, the nostril plugs system 5 comprises a two-line resilient tube 7; a plug system 8 assembled at the proximal end of the resilient tube 7 for connection of the nostril plugs system 5 to the pump system 3; and a nostril plugs assembly 9 connected to the distal end of the resilient tube 7.

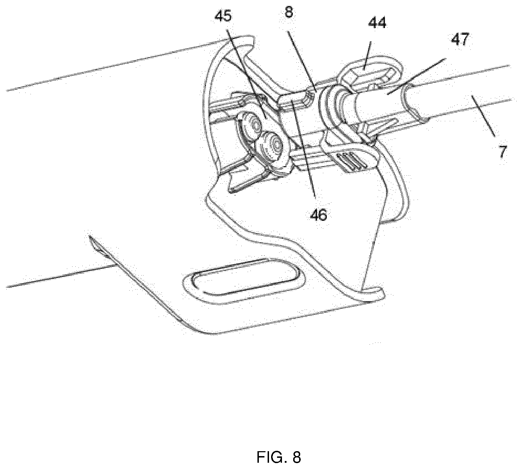

As shown in FIG. 8, the plug-in system 8 comprises two plastic components 44 that are designed to snap-fit into a socket female plug 45 of the external housing 1; a notch 46 for correct positioning of the plug-in system in the female socket plug 45 of the external housing and thus correct connection to the discharge plugs 18, 19; and a plastic accessory 47 for connection of the plug-in system to the tube 7.

The nostril plugs assembly 9 comprises a plastic accessory 39 to connect the nostril plugs assembly to the tube 7; two Y-shaped internal components 39a, 39b that distribute the liquid solution and air in a nostril plugs component 40; and a support 41 that holds all the nostril plugs assembly components together by means of a snap-fit technology and creates mechanical tightening to create an airtight chamber inside.

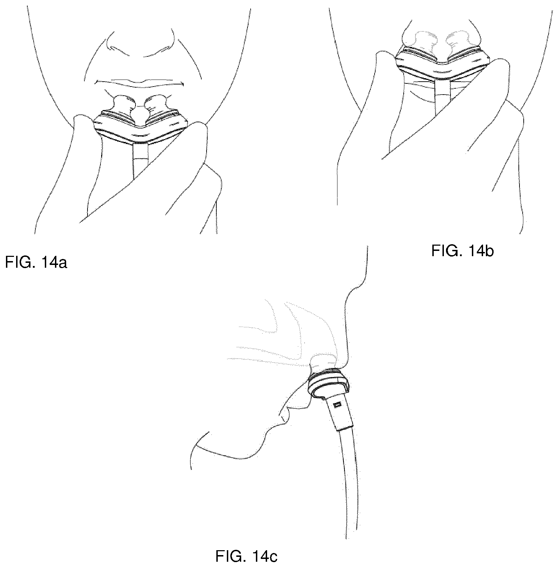

The nostril plugs component 40 has two bell-shaped expandable portions 42, 43, as shown in FIG. 10, to lock into the inner cavity as soon as they are placed on the nose. The two bell-shaped expandable portions 42, 43 are designed to have an angle α to offer perfect adaptation and ergonomics to the human nose and offer comfort and safety in the nasal cavities, so when positioned in the nostrils of a user and inflated they hold in place and the user doesn't have to hold them, as shown in FIG.14.

In a preferred embodiment of the present invention, the angle α is between 130 degrees and 140 degrees, and most preferably 135 degrees.

The two expandable bell-shaped portions 42, 43 of the nostril plugs component 40 comprise a network of internal tubes that allow the expandable portions to swell with the air coming from the air system and create an airtight lock with the user's nostrils.

The two-line resilient tube 7 has an air line 50 to inject air to the nostril plugs component 40 and a liquid line 51 to deliver the liquid solution from the cartridge 4 to the nasal cavities through an orifice 52 of the two bell-shaped expandable portions of the nostril plugs component 40.

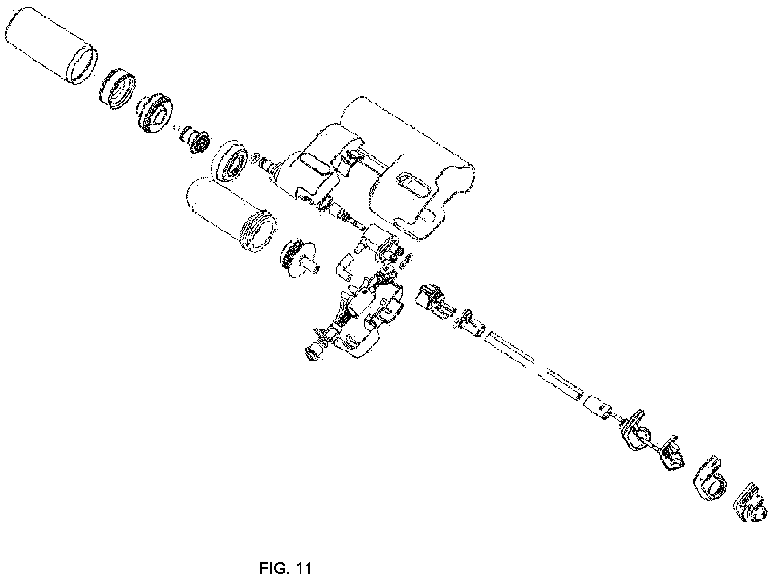

As shown in FIG. 11, the device may comprise other elements (i.e. additional sealing means) necessary for its correct operation.

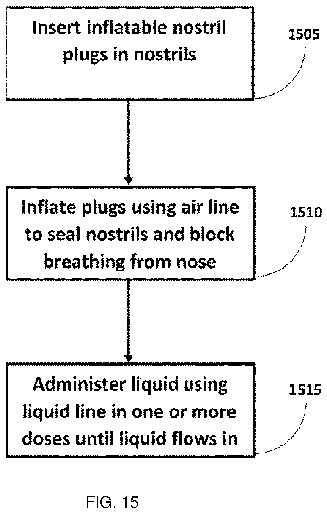

FIG. 15 is a flow chart of a method of providing intranasal liquid to nasal cavities. In a first step 1505, the nostril plugs may be inserted in the nostrils. Then, in a next step 1510, an expandable portion of each plug may be inflated by receiving air from the air system, through the air line 50, to block and/or seal each nostril, respectively, the blocking or sealing being achieved typically by expanding the expandable portion until the user is not able to inhale or exhale (breath) from the nose. The liquid line 51 may be open and may be configured to provide liquid to the interior of the nostrils. With the bilateral obstruction of the nostrils the user may still breathe from the mouth while at the same time the alar sidewalls of the nostrils may be pushed outwards by the expandable portion of the secondary channel. This may result in an expansion of the nostrils, which may free the nasal conchae and reveal the orifices of the ducts leading to the sinuses. In a next step 1515, there may be subsequent or simultaneous provision of liquid or therapeutic solution through the liquid lines 51 to both nostrils. The provision may be performed with one dose or many smaller doses so that both nostrils may be filled equally, slowly and softly to avoid irritation of the user and so that the level of the liquid in the nostrils may be maintained substantially equal all the time.

FIG. 14a schematically illustrates a user holding the nostril plugs component 40 before any liquid administration is performed. As may be seen in FIG. 14a, the plugs are arranged at an angle between them to correspond to the natural angle between the nostrils of the user. In FIG. 14b, the user has placed the plugs of the nostril plugs component 40 in the corresponding nostrils. Following said placement, air is pumped to the inflatable plugs and the nostrils are then plugged and blocked and no breathing through the nostrils can take place. The user may then let go the nostril plugs component 40 as it is held in place by the pressure exerted by the inflated plugs to the interior of the nostrils. Fig. 14c is a section view of an inclined head during the first step of an application of a method and/or device hereof. That is, the plugs have been inserted into the nostrils but no liquid has yet been provided. The head is in this implementation inclined forward to avoid passage of the liquid to the pharynx. During a next step, liquid is administered using the liquid line until the level of the provided solution reaches slowly to the plane of the soft palate. Then the reflex of swallowing may be triggered. The reflex movement of swallowing may be complete due to the stimulation of the soft palate by the intranasal liquid or therapeutic solution even though there is no bolus in the mouth for swallowing. To perform the swallowing reflex the soft palate may move upwards and frontwards. The available space may thus be reduced and the level of the liquid or solution may be raised and cover the orifices of the ducts of the paranasal sinuses. At the same time, the forward movement of the soft palate may push air towards the surface of the solution. Consequently, pressure may be exerted on the solution to push it towards the nostrils' openings. Because the nostrils' openings are blocked by the intranasal parts of the device, the solution may seek escape through the orifices of the ducts of the paranasal sinuses. In repetition of the swallowing movement the liquid may find an escape route through the paranasal sinuses' ducts, while the device may slowly replenish the quantity of the solution so that the level of the solution remains at the level of the soft palate and maintains the stimulation of the soft palate. In some non-limiting implementations, the whole process, blocking of the nostrils, expansion of the alar sidewalls of the nostrils, revealing of the duct orifices of both nostrils with the expanding intranasal portions of the device, the simultaneous provision of solution to both nostrils, the stimulation or triggering of the automatic reflex of swallowing, the pressure to the surface of the solution from the forward moving air caused by the upward movement of the soft palate and the introduction of the solution to the paranasal sinuses-a process performed slowly and softly, at a pace acceptable by any user even by small children-provides a complete solution.

The perfectly tolerable use of the device may give the user the possibility to use the therapeutic process with ease, even when at work or while sitting on a couch (e.g. while watching TV), for as long as a user wants, providing an effective method. The friendliness of a prolonged therapeutic use provides the necessary time for the therapeutic solution to act during a necessary time to maximize the therapeutic result in the nasal and paranasal chambers. The time may range between five (5) and fifteen (15) minutes. However, in some cases even longer periods of half an hour may be beneficial so that the therapeutic effect of the liquid may take place.

The end result may provide an effective solution desired and long sought after by the medical community and users. It may be made possible by the combination of characteristics of the proposed devices and/or methods. That is, the devices may provide a complete sealing of the nostrils, in some implementations thus provoking the swallowing reflex when sufficient liquid is introduced in the nostrils.

Furthermore, the way that the nasal cavities are sealed may not be a simple sealing but may produce some further effects: The expansion of the intranasal part of the device in the nostril may be disposed to push outwards the alar sidewalls of the nostrils which results in a widening of the nasal vestibule, and in some implementations thus freeing the nasal conchae and revealing the ducts of the paranasal sinuses which may facilitate the introduction of liquid to the paranasal sinuses.

Moreover, proper obstruction of both nostrils provided by the use of the proposed devices and/or methods, may allow the solution to remain in the nostrils and in the paranasal sinuses for as long as the user may wish, thereby providing the necessary time so that the therapeutic and/or cell-regenerative and/or healing properties of the medicine or of the saline (sea water) or of the extracts of aromatic plants to give results.

Therefore, the therapeutic result for the user or patient may depend on the proper application of the process that is provided by the proposed devices. It is also mentioned that an application of the solution to the nostrils may be performed in a soft and totally controllable manner and in small doses. This may in some applications be achieved by the cartridge construction that may provide solution under pressure in such a way that with each application (pressure) only a small controlled quantity may be provided to both nostrils.

While the examples have been described in detail in the foregoing description, the same is to be considered illustrative and not restrictive in character, being understood that only some examples have been shown and described and that all changes and modifications that come within the spirit of the examples are desired to be protected. While said particular examples of the present disclosure have been described, it would be obvious to those skilled in the art that various other changes and modifications may be made without departing from the spirit and scope of the disclosure. It is therefore intended to cover in the appended claims all such changes and modifications are within the scope of the disclosure.

### REFERENCES

**1.** Lirong Zou et al. SARS-CoV-2 Viral Load in Upper Respiratory Specimens of Infected Patients, N Engl J Med 2020; 382:1177-1179, DOI: 10.1056/NEJMc2001737.
**2.** Gengler I, Wang JC, Speth MM, Sedaghat AR. Sinonasal pathophysiology of SARS-CoV-2 and COVID-19: A systematic review of the current evidence. Laryngoscope Investigative Otolaryngology. 2020;1-6. https:// doi.org/10.1002/lio2.384.
**3.** Rafael R. G., Machado, Talita Glaser, Danielle B. Araujo, Lyvia Lintzmaier Petiz, Danielle B. L. Oliveira, Giuliana S. Durigon, Alessandra L. Leal, João Renato R. Pinho, Luis Carlos S. Ferreira, Henning Ulrich, Edison L. Durigon, Cristiane R. Guzzo , Hypertonic saline solution inhibits SARS-CoV-2 in vitro assay. BioRχiv, doi: https://doi.org/10.1101/2020.08.04.235549
**4.** Sandeep Ramalingam, Baiyi Cai, Junsheng Wong, Matthew Twomey, Rui Chen, Rebecca M. Fu, Toby Boote, Hugh McCaughan, Samantha J. Griffiths, Jürgen G. Haas, Antiviral innate immune response in non-myeloid cells is augmented by chloride ions via an increase in intracellular hypochlorous acid level, Scientific Reports (2018) 8:13630 | DOI:10.1038/s41598-018-31936-y (www.nature.com/scientificreports).
**5.** Sandeep Ramalingam, Catriona Graham, Jenny Dove, Lynn Morrice, Aziz Sheikh, A pilot, open labelled, randomised controlled trial of hypertonic saline nasal irrigation and gargling for the common cold, Scientific Reports (2019) 9:1015 https://doi.org/10.1038/s41598-018-37703-3 (www.nature.com/scientificreports).

## Claims

1. A device for controlled administration of a liquid solution to nasal cavities and paranasal sinuses of a user, comprising:
- an external housing (1) defining an inner space and having a proximal end and a distal end, the external housing comprising:
- an air system (2) for injecting air to a nostril plugs assembly (9);
- a pump system (3) for pumping the liquid solution to the nasal cavities and connected to the air system (2) by means of a resilient tube (6);
- a cartridge (4) holding the liquid solution, removably connected to the pump system (3) at the proximal end of the external housing (1); and
- a nostril plugs system (5) removably connected to the pump system (3) at the distal end of the external housing (1) and comprising:
- a two-line resillient tube (7) having a proximal end and a distal end;
- a plug-in system (8) connected to the proximal end of the resilient tube (7); and
- a nostril plugs assembly (9) connected to the distal end of the resilient tube (7).

2. The device according to claim 1, wherein the air system (2) comprises:
- an air bulb (20);
- an air release valve (21) connected to the air bulb (20) through an air bulb base (22) and comprising:
- a valve cover (23);
- a valve plunger (24);
- a valve cover sealing means (25);
- a valve plunger sealing means (26)
- a first spring means (27) that applies a constant pressure holding the valve plunger sealing means (26) and allows air flow in only one direction, preventing reverse flow;
- an actuator (28) activated by the user's pressure and comprising and an actuator sealing means (29);
- a second spring means (30); and
- an air outlet (31) for connection to the silicone tube (6).

3. The device according to claim 1, wherein the pump system (3) comprises:
- a cylinder (10);
- a spring means (11) arranged around the outside of the cylinder (10);
- a pump nozzle (12) for connection with the cartridge (4) comprising sealing means (13);
- a hollow piston (14) which is movable in the cylinder (10) and comprises sealing means (13);
- a valve member (15) which is guided by the hollow piston and is mounted so as to be axially movable with the piston (14); and
- a pump base (16) comprising an air inlet port (17) connected to an air discharge plug (18) to channel the air coming from the air system (2) and a liquid discharge plug (19) to channel the liquid from the cartridge (4).

4. The device according to claim 1, wherein the external housing (1) is made of a plastic material.

5. The device according to previous claims, wherein the external housing (1) holds the pump system (2) and the air system (3) by means of a snap-fit mechanism (48) through a subframe set (49).

6. The device according to previous claims, wherein the cartridge (4) comprises:
- a container (32) comprising the liquid solution;
- a hollow cylindrical piston (33) arranged inside the container (32);
- a one-way valve mechanism (34);
- a container lid (35);
- a pump connection mechanism (36); and
- a cartridge lid (37) to lock the container (32).

7. The device according to claim 6, wherein the container (32) has a content of 50ml of liquid solution.

8. The device according to claim 6, wherein the cartridge lid (37) comprises a safety mechanism (38) to protect the connection area of the cartridge from dirt and to certify that the cartridge has not been used before.

9. The device according to previous claims, wherein the two-line silicone tube (7) has an air line (50) to inject air to the nostril plugs assembly (9) and a liquid line (51) to deliver the liquid solution from the cartridge (4) to the nasal cavities.

10. The device according to previous claims, wherein the plug-in system (8) comprises:
- two plastic components (44) that are designed to snap-fit into a socket female plug (45) of the external housing (1);
- a notch (46) for correct positioning of the plug system in the liquid and air discharge plugs (18, 19); and
- a plastic accessory (47) for connection of the plug-in system to the silicone tube (7).

11. The device according to previous claims, wherein the nostril plugs assembly (9) comprises:
- a plastic accessory (39) to connect the nostril plugs assembly to the silicone tube (7);
- a nostril plugs component (40);
- two Y-shaped internal components (39a, 39b) that distribute the liquid solution and air in the nostril plugs component (40); and
- a support (41) that holds all the nostril plugs assembly components together by means of a snap-fit technology or ultrasonic bonding and creates mechanical tightening to create an airtight chamber inside.

12. The device according to previous claims, wherein the nostril plugs component (40) has two bell-shaped expandable portions (42, 43).

13. The device according to claim 12, wherein the two bell-shaped expandable portions (42, 43) are arranged at an angle (α) to offer perfect adaptation and ergonomics to the human nose and offer comfort and safety in the nasal cavity, the angle α being between 130 degrees and 140 degrees, most preferably 135 degrees.

14. The device according to previous claims, wherein the two expandable bell-shaped portions (42, 43) of the nostril plugs component (40) comprise a network of internal tubes that allow the expandable portions to swell with the air coming from the air system and create an airtight lock with the user's nostrils.

15. A cartridge holding a liquid solution for connection to a pump system of a device for controlled administration of a liquid solution to nasal cavities and paranasal sinuses of a user, comprising:
- a container (32) comprising the liquid solution;
- a hollow cylindrical piston (33) arranged inside the container (32);
- a one-way valve mechanism (34);
- a container lid (35);
- a pump connection mechanism (36); and
- a cartridge lid (37) to lock the container (32): and
- a safety mechanism (38) to protect the connection area of the cartridge from dirt and to certify that the cartridge has not been used before.
